# EUROPÄISCHE PATENTSCHRIFT

(11) **EP 1 081 172 B1**
(45) Veröffentlichungstag und Bekanntmachung des Hinweises auf die Patenterteilung: **21.05.2003**
(21) Anmeldenummer: 00117886.2
(22) Anmeldetag: 19.08.2000
(51) Int. Cl.: C08G 63/695, C08G 63/688, A61K 7/06

(54) **Sulfonierte Kammpolymere mit ausgewähltem Lithium/Natrium-Verhältnis und Zubereitungen, insbesondere haarkosmetische Zubereitungen auf der Grundlage von solchen sulfonierten Kammpolymeren**
Sulfonated comb polymers and preparations with selected Lithium/Sodium-ratio, especially hair cosmetic preparations based on such sulfonated comb polymers
Polymères sulfonés ramifiés en forme de peigne ayant un rapport de Lithium/Sodium selectionné et préparations, en particulier préparations cosmétiques capillaires à base de tels polymères sulfonés ramifiés en forme de peigne

(30) Priorität: 04.09.1999 DE 19942302
(43) Veröffentlichungstag der Anmeldung: 07.03.2001
(73) Patentinhaber: Beiersdorf AG, 20253 Hamburg (DE)
(72) Erfinder: Koller, Andreas, Dr., 21035 Hamburg (DE); Detert, Marion, 22455 Hamburg (DE)

(56) Entgegenhaltungen:
- EP-A- 0 204 234
- EP-A- 1 043 352
- EP-A- 1 048 287
- WO-A-99/45055
- DATABASE WPI Section Ch, Week 198506 Derwent Publications Ltd., London, GB; Class A25, AN 1985-035383 XP002148564 & JP 59 230057 A (TOA GOSEI CHEM IND LTD), 24. Dezember 1984 (1984-12-24)

## Beschreibung

Die vorliegende Erfindung betrifft neue sulfonierte Kammpolymere und Zubereitungen, solche sulfonierte Kammpolymere enthaltend. Insbesondere betrifft die vorliegende Erfindung haarkosmetische Wirkstoffe und Zubereitungen zur Festigung, Formgebung, Kräftigung und Strukturverbesserung der Haare.

Eine ansprechend aussehende Frisur wird heute allgemein als unverzichtbarer Teil eines gepflegten Äußeren angesehen. Dabei gelten aufgrund aktueller Modeströmungen immer wieder Frisuren als chic, die sich bei vielen Haartypen nur unter Verwendung bestimmter festigender Wirkstoffe aufbauen bzw. für einen längeren Zeitraum aufrechterhalten lassen.

Aus diesen Gründen werden seit geraumer Zeit teils Haarpflegekosmetika verwendet, welche dazu bestimmt sind, nach Einwirken aus dem Haar wieder ausgespült zu werden, teils solche, welche auf dem Haar verbleiben sollen. Letztere können so formuliert werden, daß sie nicht nur der Pflege des einzelnen Haars dienen, sondern auch das Aussehen der Haartracht insgesamt verbessern, beispielsweise dadurch, daß sie dem Haar mehr Fülle verleihen, die Haartracht über einen längeren Zeitraum fixieren oder seine Frisierbarkeit verbessern.

Die Eigenschaft der Fülle wird einer Frisur beispielsweise zugeschrieben, wenn das Haar nach der Behandlung nicht flach auf der Kopfhaut aufliegt und gut frisierbar ist.

Die Eigenschaft des Volumens wird einer Frisur beispielsweise zugeschrieben, wenn das Haar nach der Behandlung Fülle und Sprungkraft aufweist.

Die Eigenschaft des Body's wird einer Frisur beispielsweise zugeschrieben, wenn das Haarvolumen selbst unter äußeren, störenden Einflüssen groß bleibt

Festigende Wirkstoffe, bei denen es sich in der Regel um polymere Verbindungen handelt, können in übliche Haarreinigungs- oder -konditioniermittel eingearbeitet werden. In vielen Fällen ist es aber vorteilhaft, sie in Form spezieller Mittel wie Haarfestiger oder Haarsprays anzuwenden.

Es gibt nun in jüngster Zeit eine Reihe von Entwicklungen auf dem Haarkosmetikgebiet, die einen Bedarf an neuartigen festigenden Wirkstoffen bzw. neuen Formulierungsformen geweckt haben. Viele dieser Entwicklungen beruhen dabei nicht auf anwendungstechnischen Nachteilen oder Unzulänglichkeiten der bekannten Mittel, sondern z.B. auf Umweltschutz-Gesichtspunkten, gesetzlichen Auflagen oder anderen "nichttechnischen" Ursachen.

So wird insbesondere verstärkt ein Übergang von Mitteln auf Basis flüchtiger organischer Verbindungen (sogenannter volatile organic compounds" oder auch kurz: VOC's), z.B. Alkoholen, zu Mitteln auf wäßriger Basis angestrebt.

Der Stand der Technik läßt es aber an Wirkstoffen (Polymeren) und Zubereitungen mangeln, welche den vorab genannten Anforderungen entsprechen. Die Haartracht fixierende Zubereitungen des Standes der Technik enthalten beispielsweise in der Regel Bestandteile (synthetische oder natürliche Polymere), welche Gefahr laufen, bei teilweisen oder vollständigen Ersatz leichtflüchtiger organische Bestandteile durch Wasser eine signifikante Beeinträchtigung der Produkteigenschaften zu erfahren, was oft durch geschickte Formulierung kompensiert werden muß. Zudem zeichnen sich die fixierenden Zubereitungen des Standes der Technik häufig durch nur schwierig bzw. aufwendig zu formulierende Rezepturbestandteile mit ungenügender Langzeitstabilität aus, wobei dieses besonders auf Siliconderivate zutrifft, die zur Verbesserung der Flexibilität und Taktilität der Polymerfilmoberfläche eingesetzt werden.

Es bestand also die Aufgabe, entsprechende Mittel zu entwickeln, die hinsichtlich der anwendungstechnischen Eigenschaften, beispielsweise dem Sprühverhalten und der Trocknungszeit bei Haarsprays, die vom Verbraucher gesteckten Erwartungen erfüllen und gleichzeitig einen reduzierten Anteil an flüchtigen organischen Verbindungen aufweisen, ohne daß die elementaren Eigenschaften des Polymerfilms auf den Haaren, wie z.B. Klarheit/Transparenz, Oberflächentaktilität, Glanz, Elastizität und Auswaschbarkeit negativ beeinflußt werden und die Verarbeitbarkeit der Formulierungsbestandteile einfach und unproblematisch ist.

Es wurde nun gefunden, und darin liegt die Lösung der Aufgaben begründet, daß wasserlösliche und/oder wasserdispergierbare Kammpolymere, bestehend aus einer Polymerhauptkette und mit dieser Polymerhauptkette über Estergruppen verknüpften sulfongruppenhaltigen Polyesterseitenarmen, welche wenigstens teilweise durch Natrium und Lithiumgegenionen neutralisiert wurden, wobei das molare Verhältnis von Lithium zu Natrium zwischen 0,1 und 50, bevorzugt zwischen 0,5 und 25, liegt, die Nachteile des Standes der Technik beseitigen oder zumindest mindern.

Die erfindungsgemäßen Kammpolymere zeichnen sich sowohl durch gute Wasser- und Alkoholverträglichkeit als auch durch günstige Filmeigenschaften und hohem Netzvermögen aus. Zudem sind sie einfach zu formulieren.

Die Grundstruktur der erfindungsgemäßen Kammpolymere folgt im wesentlichen dem folgenden Schema:

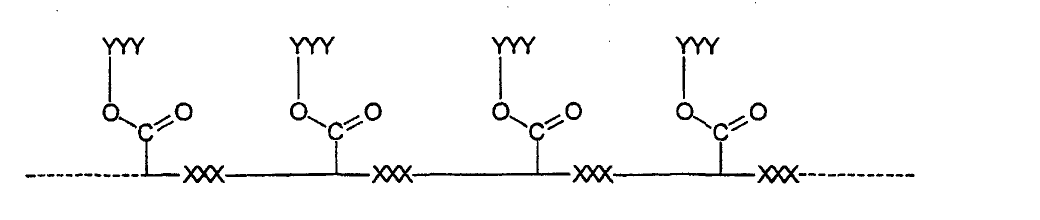

Dabei bedeuten die miteinander verbundenen Gruppierungen mit der Bezeichnung XXX den Grundkörper eines Polymerrückgrates, an welchem über Esterfunktionen Molekütgruppierungen verbunden sind, welche die Bezeichnung YYY tragen. Die Molekülgruppierungen YYY stellen sowohl die vollständigen sulfongruppenhaltigen Polyesterseitenarme der erfindungsgemäßen Kammpolymere dar, können aber auch andere Molekülgruppierungen darstellen.

Dabei besteht die polymere Hauptkette der erfindungsgemäß eingesetzten Kammpolymere bevorzugt aus:
a) polymeren aliphatischen, cycloaliphatischen oder aromatischen Polycarbonsäuren bzw. deren Derivaten wie beispielsweise Polyacrylsäure, Polymethacrylsäure und deren Ester (Ester der beiden Säuren mit aliphatischen, cycloaliphatischen oder aromatischen Alkoholen mit C₁ bis C₂₂), Maleinsäure, Maleinsäureanhydrid, Fumarsäure und Polynorbonensäure. Die mittleren Molekulargewichte der eingesetzten Polycarbonsäure können zwischen 200 und 2.000.000 g/mol liegen wobei der Bereich von 2.000 - 100.000 g/mol bevorzugt Verwendung findet.

Die mittleren Molekulargewichte der eingesetzten Polycarbonsäure können zwischen 200 und 2.000.000 g/mol liegen wobei der Bereich von 2.000 -100.000 g/mol bevorzugt Verwendung findet.

Die Anbindung der Polyester-Seitenketten erfolgt über eine Estergruppe, die durch die Reaktion einer funktionellen Gruppe der Hauptkette (-COOH im Falle der Polycarbonsäuren oder -OH im Falle der Polyalkohole) mit einer entsprechenden Gruppe des Polyesters (OH im Falle der Polycarbonsäuren und COOH im Falle der Polyalkohole). Selbstverständlich können auch reaktive Derivate der eben angeführten Komponenten zur Reaktion gebracht werden (beispielsweise Anhydride, Ester, Halogenverbindungen und dergleichen mehr).

Die erfindungsgemäß eingesetzten Polyester können sich vorteilhaft durch folgende generische Strukturformeln auszeichnen: 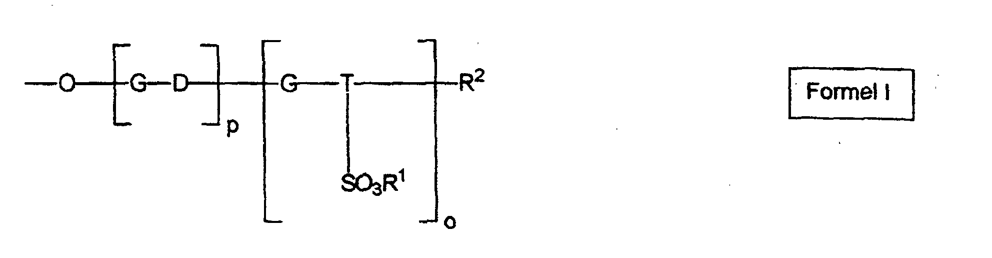 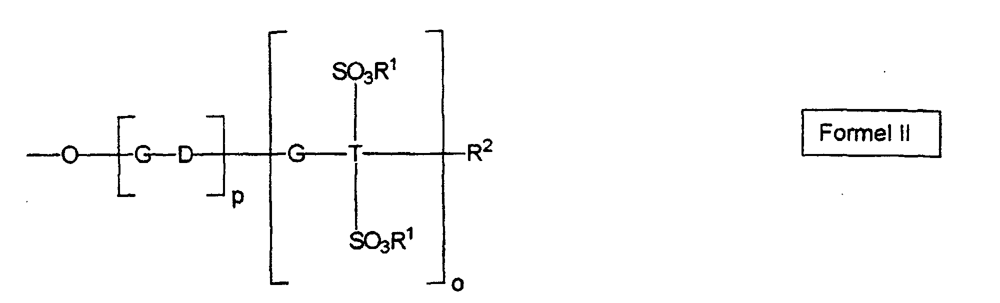 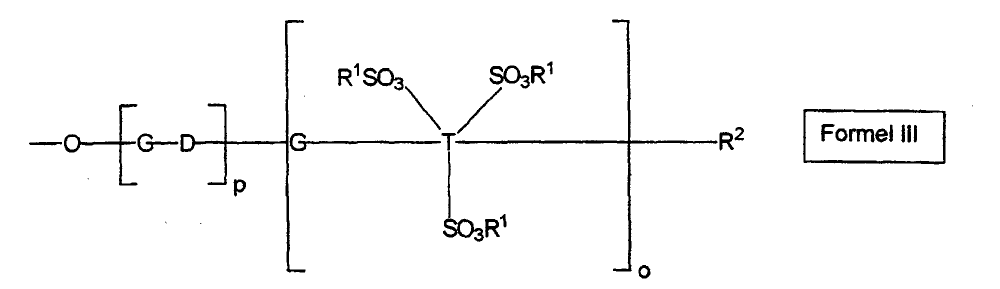 usw.

Dabei können p und o so gewählt werden, daß die vorab bezeichneten mittleren Molekulargewichte der eingesetzten Hauptkettenbestandteile zuwege kommen.

Die Polyester-Seitenketten gemäß Formel I - III bestehen vorteilhaft aus:
- G :: einer mindestens zwei endständige Sauerstoffatome enthaltende aromatische, aliphatische oder cycloaliphatische Organyleinheiten mit einer Kohlenstoffzahl von C₂ bis C₂₂ oder Abkömmlinge eines Polyglykols der Form HO-[R³-O)ₖ-[R⁴-O)ₘ-H, entsprechend einer Organyleinheit 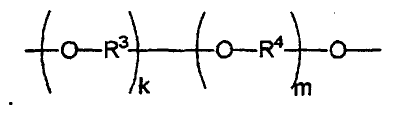
Die Reste R³ und R⁴ stellen Alkylenreste dar mit einer Kohlenstoffzahl von C₂-C₂₂, wobei beide Reste nicht notwendigerweise verschieden sein müssen.

Für die Koëffizienten k und m gilt: k+m ≥ 1, wobei k und m ferner so gewählt werden können, daß die vorab bezeichneten mittleren Molekulargewichte der eingesetzten Hauptkettenbestandteile zuwege kommen.
- D :: einer mindestens zwei endständige Acylgruppen enthaltenden aromatischen, aliphatischen oder cycloaliphatischen Organyleinheit mit einer Kohlenstoffzahl von C₂ bis C₂₂, wobei auch Kombinationen aus mehreren verschiedenen Säurekomponenten im beanspruchten Zielmolekül enthalten sein können, beispielsweise eine Organyleinheit des Schemas 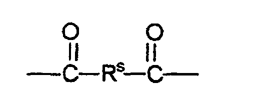
wobei R^{s} aromatische und lineare oder cyclische, gesättigte oder ungesättigte aliphatische bifunktionale Reste mit Kohlenstoffzahlen von C₂ bis C₂₂ darstellen kann.
- T:: eine Verbindung aus der Gruppe der mindestens zwei endständige Acylgruppen enthaltenden sulfonierten aromatischen, aliphatischen oder cycloaliphatischen Organylverbindungen
- R¹:: Lithium und/oder Natrium neben gegebenenfalls weiteren Gegenionen, z.B., Kalium, Magnesium, Calcium, Ammonium, Monoalkylammonium, Dialkylammonium, Trialkylammonium oder Tetraalkylammonium bedeuten kann, worin die Alkylpositionen der Amine unabhängig voneinander mit C₁ bis C₂₂-Alkylresten und 0 bis 3 Hydroxylgruppen besetzt sind.
- R²:: einen Molekülrest, gewählt aus den Gruppen der

- aromatischen, aliphatischen oder cycloaliphatischen Aminofunktionen: ( -NH-R⁵, -NR⁵₂ wobei R⁵ einen Alkyl- oder Arylrest mit C₁ bis C₂₂ darstellen kann)
- aromatischen, aliphatischen oder cycloaliphatischen Monocarbonsäuregruppen: (-COOR⁶ wobei R⁶ ein Alkyl- oder Arylrest darstellt mit C₁ bis C₂₀₀)
- über Etherfunktionen verbrückten aromatischen, aliphatischen oder cycloaliphatischen Organylreste: (-O-R⁵)
- über Etherfunktionen verbrückenden Polyalkoxyverbindungen der Form

   -O-[R⁷-O]_{q}-[R⁸-O]ᵣ-Y

   Die Reste R⁷ und R⁸ stellen vorteilhaft Alkylenreste dar mit einer Kohlenstoffzahl von C₂-C₂₂, wobei beide Reste nicht notwendigerweise verschieden sein müssen.
   Der Rest Y kann sowohl Wasserstoff als auch aliphatischer Natur mit C₁-C₂₂ sein. Für die Koeffizienten q und r gilt: q+r ≥ 1.
- über Etherfunktionen verbrückenden einfach oder mehrfach ethoxylierten sulfonierten Organylreste oder bevorzugt deren Alkali- oder Erdalkalisalze, wie beispielsweise vorteilhaft gekennzeichnet durch die generische Strukturformel

   -(O-CH₂-CH₂)ₛ-SO₃R¹

   mit s ≥ 1, und wobei s ferner so gewählt werden kann, daß die vorab bezeichneten mittleren Molekulargewichte der eingesetzten Hauptkettenbestandteile zuwege kommen.

Die Funktionalität der erfindungsgemäß eingesetzten Komponenten beschränkt sich natürlich nicht auf die Verwendung von OH-Gruppierungen, sondern schließt auch COOH-Endgruppierungen ein oder Mischungen von beiden, wobei auch hier gilt, daß mindestens zwei COOH-Gruppen frei im Molekül vorhanden sein müssen. Reaktive Derivate wie Anhydride, Ester, Epoxide oder Halogenide sind natürlich ebenfalls einsetzbar.

Die mittleren Molekulargewichte der erfindungsgemäßen Kammpolymere können vorteilhaft zwischen 200 und 2.000.000 g/mol liegen, besonders vorteilhaft zwischen 200 und 100.000 g/mol liegen wobei der Bereich von 1.000 - 30.000 g/mol bevorzugt Verwendung findet, ganz besonders vorteilhaft von 5.000 - 15.000 g/mol.

Die erfindungsgemäßen Polyester werden vorteilhaft hergestellt durch Veresterung oder Umesterung der zugrundeliegenden funktionellen Alkoholkomponenten und Diolen mit den Carbonsäuren bzw. deren geeigneten Derivate (beispielsweise Alkylester, Halogenide und dergleichen mehr) in Gegenwart eines Veresterungskatalysators wie Alkalimetallhydroxide, deren -carbonate und Acetate, Erdalkalimetalloxide, -hydroxide, -carbonate und -acetate sowie Alkalimetall- und Erdalkalimetallsalze von Fettsäuren mit 6 bis 22 Kohlenstoffatomen. Weiterhin kommen Titanverbindungen, wie Titanate, metallisches Zinn und organische Zinnverbindungen, wie Mono- und Dialkylzinnderivate als Veresterungskatalysatoren in Betracht. Vorzugsweise wird die Veresterung/Umesterung unter Verwendung von Zinnschliff oder Titantetraisopropylat als Katalysator durchgeführt.

Die Veresterung/Umesterung wird bevorzugt bei Temperaturen von 120 °C bis 280 °C durchgeführt, wobei der entstehende leichter siedende Kondensat (Alkohole oder Wasser) destillativ aus dem Kondensationsprodukt entfernt wird, bevorzugt unter vermindertem Druck bis zu < 0,1 mbar.

Als Edukte für das Polyestergerüst erfindungsgemäßer Kammpolymere können polymere aliphatische, cycloaliphatische oder aromatische Polycarbonsäuren bzw. deren Derivate wie beispielsweise Polyacrylsäure, Polymethacrylsäure und deren Ester (Ester der beiden Säuren mit aliphatischen, cycloaliphatischen oder aromatischen Alkoholen mit C₁ bis C₂₂), Maleinsäure, Maleinsäureanhydrid, Fumarsäure und Polynorbomensäure eingesetzt werden. Die mittleren Molekulargewichte der einzelnen Polycarbonsäuren können zwischen 200 und 2.000.000 g/mol liegen, wobei der Bereich von 2.000 -100.000 g/mol bevorzugt Verwendung findet.

Auch statistische oder blockartige Copolymere der oben genannten Verbindungsklasse mit anderen vinylischen Monomeren wie beispielsweise Styrol, Acrylamid, α-Methylstyrol, Styrol, N-Vinylpyrrolidon, N-Vinylcaprolacton, Acrylamidopropylensulfonsäure und deren Alkal-, Erdalkali- und Ammoniumsalze, MAPTAC (Methacrylamidopropyltrimethylammoniumchlorid), DADMAC, Vinylsulfonsäure, Vinylphosphonsäure, Crotonsäure, Vinylacetamid, Vinylmethylacetamid, Vinylforamid, Acrylsäure oder Methacrylsäurederivate (beispielsweise freie Säure oder Ester), oder Acrylamidderivate oder Vinylacetat können zur Ausbildung der polymeren Hauptkette dienen.

Als Basis für mindestens zwei endständige Sauerstoffatome enthaltende aromatische, aliphatische oder cycloaliphatische Organyleinheiten mit einer Kohlenstoffzahl von C₂ bis C₂₂ oder Abkömmlinge eines Polyglykols der Form HO-[R³-O)ₖ-[R⁴-O)ₘ-H, können bifunktionelle Alkoholkomponenten eingesetzt werden.

Dafür eignen sich insbesondere mindestens difunktionelle aromatische, aliphatische oder cycloaliphatische Alkohole mit einer Kohlenstoffzahl von C₂ bis C₂₂ oder ein Polyglycol der Form HO-[R³-O]ₖ-[R⁴-O]ₘ-H. Die Reste R³ und R⁴ stellen Alkylreste dar mit einer Kohlenstoffzahl von C₂ bis C₂₂, wobei beide Reste gleich oder verschieden sein können. Für die Koeffizienten k und m gilt: k+m ≥ 1, wobei k und m ferner so gewählt werden können, daß die vorab bezeichneten mittleren Molekulargewichte der eingesetzten Hauptkettenbestandteile zuwege kommen..

Es kann von besonderem Vorteil sein, statt difunktioneller Alkoholkomponenten tri-, tetra- oder allgemein polyfunktionelle Alkoholkomponenten einzusetzen, beispielsweise vorteilhaft gewählt aus der folgenden Gruppe:

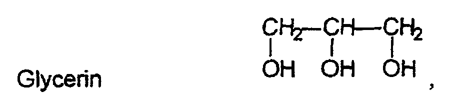

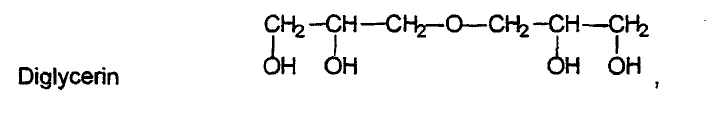

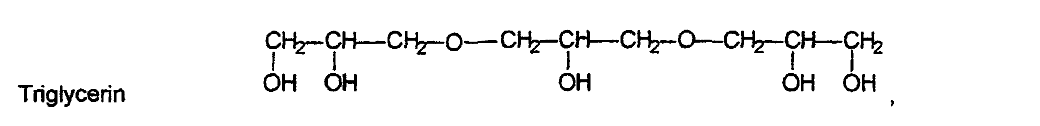

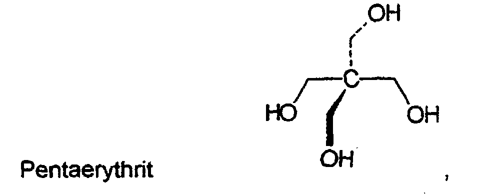

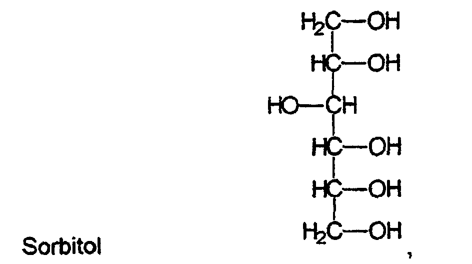

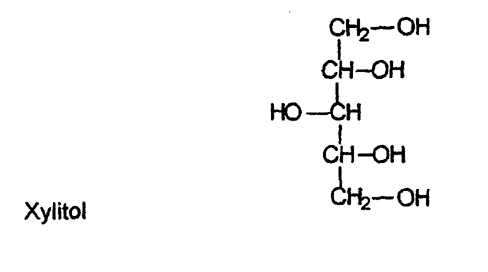

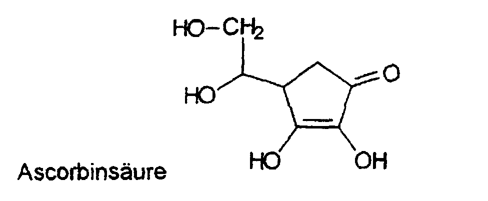

Als Basis für mindestens zwei endständige Acylgruppen enthaltende aromatische, aliphatische oder cycloaliphatische Organyleinheiten mit einer Kohlenstoffzahl von C₂ bis C₂₂, beispielsweise Organyleinheiten des Schemas 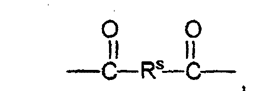 können beispielsweise aromatische und lineare oder cyclische, gesättigte oder ungesättigte aliphatische Carbonsäuren mit einer Kohlenstoffzahl von C₂ bis C₂₂ oder dessen Anhydride eingesetzt werden, beispielsweise Phthalsäure, Isophthalsäure, Naphthalindicarbonsäure, Cyclohexandicarbonsäure, Adipinsäure, Bernsteinsäure, Glutarsäure, Pimelinsäure, Korksäure, Azelainsäure, Sebacinsäure, Brassylsäure eingesetzt werden. Auch Kombinationen aus mehreren verschiedenen Säurekomponenten sind als Monomereinheit im beanspruchten Zielmolekül möglich.

Als sulfongruppenhaltige Monomere eignen sich sulfonierte aromatische, aliphatische oder cycloaliphatische Dialkohole, Disäuren bzw. deren Ester, wie beispielsweise Sulfobemsteinsäure, 5-Sulfoisophthalsäure oder deren Alkali- oder Erdalkalisalze oder Mono-, Di-, Tri- oder Tetraalkylammoniumsalze mit C₁ bis C₂₂-Alkylresten. Unter den Alkalisalzen sind insbesondere Lithium- und Natriumsalze bevorzugt.

Weiterhin kommen aromatische, aliphatische oder cycloaliphatische Amine mit C₁ bis C₂₂ Alkyl- bzw. Arylresten und/oder aromatische, aliphatische oder cycloaliphatische Monocarbonsäuren mit C₁ bis C₂₀₀ Alkyl- oder Arylresten und/oder Polyalkoxyverbindungen der Form -O-[R⁷-O]_{q}-[R⁸-O]ᵣ-X, wobei die Reste R⁷ und R⁸ Alkylreste, die gleich oder verschieden sein können eine Kohlenstoffzahl von C₂ bis C₂₂ darstellen und der Rest X sowohl Wasserstoff als auch aliphatischer Natur mit C₁ -C₂₂ sein kann und die Koeffizienten q und r: q+r ≥ 1 sind, zum Einsatz.

Ebenso geeignet sind sulfonierte Mono- oder Polyethylenglykole oder bevorzugt deren Alkali- oder Erdalkalisalze: (H-(O-CH₂-CH₂)ₛ-SO₃R¹ mit s ≥ 1, wobei s ferner so gewählt werden kann, daß die vorab bezeichneten mittleren Molekulargewichte der eingesetzten Hauptkettenbestandteile zuwege kommen.)

Zur Herstellung der erfindungsgemäßen Polyester werden die zur Ausbildung der Seitenkette eingesetzten Alkohole und Säuren bzw. Ester vorteilhaft in den molaren Verhältnissen von 1:1 bis etwa 10:1 (1 bzw. 10 Teile Di- oder Polyol) eingesetzt und der sich bildende Alkohol und Wasser und die Überschußkomponente nach erfolgter Kondensation destillativ entfernt. Im Zielmolekül liegen Alkohol- und Säurekomponenten vorzugsweise im ungefähren stöchiometrischen Verhältnis 1:1 vor.

Der Anteil der sulfonsäureresthaltigen Säurekomponenten beträgt 1 bis 99 mol.-%, bevorzugt 10 bis 40 besonders bevorzugt 15 bis 25 mol.-% bezogen auf die Gesamtmenge an Carbonsäuren.

Sehr günstige anwendungstechnische Eigenschaften haben die sulfongruppenhaltigen Polyester der allgemeinen Formel I, wenn als Diolkomponenten 1,2-Propandiol und/oder Diethylenglycol und/oder Cyclohexandimethanol, als Carbonsäuren Isophthalsäure auch mit,1,3-Cyclohexandicarbonsäure oder auch mit 2,6-Naphthalindicarbonsäure oder auch mit Adipinsäure und als sulfogruppenhaltige Reste 5-Sulfoisophthalsäure-Natrium- und/oder Lithiumsalz, das Natrium- und/oder Lithiumsalz der Isethionsäure eingesetzt werden.

Nachfolgend ist ein Ausschnitt aus einem erfindungsgemäßen Kammpolymermolekül aufgeführt, wobei eine Polyacrylsäurekette das Rückgrat des Kammpolymermoleküls bildet. Die Säurefunktionen sind mit Polyolen verestert, welche ihrerseits mit einer Säurefunktion von Isophthalsäuremolekülen verestert sind. Weitere Polyole, von denen sich Strukturelemente dieses Polymermoleküls herleiten sind Pentaerythritol, 1,2-Propandiol. Als sulfonatgruppenhaltiges Agens, von dem sich Strukturelemente des Polymermoleküls herleiten, dient beispielsweise das 5-Sulfoisophthalsäuredialkylester-Natrium- und/oder Lithiumsalz.

Aus Gründen der Reaktionsführung, welche dem Fachmann bekannt sind, herrscht im Zielpolymer keine absolute Einförmigkeit der Substitution vor, vielmehr ist von einer gewissen statistischen Verteilungsbreite der Substitution auszugehen. Femer werden bestimmte reaktive Molekülgruppierungen auch zu Vernetzung zweier oder mehrerer Polymerketten zu einem mehr oder weniger komplexen Netzwerk zu beobachten sein, wie es das nachfolgende Molekülschema auch darzustellen versucht.

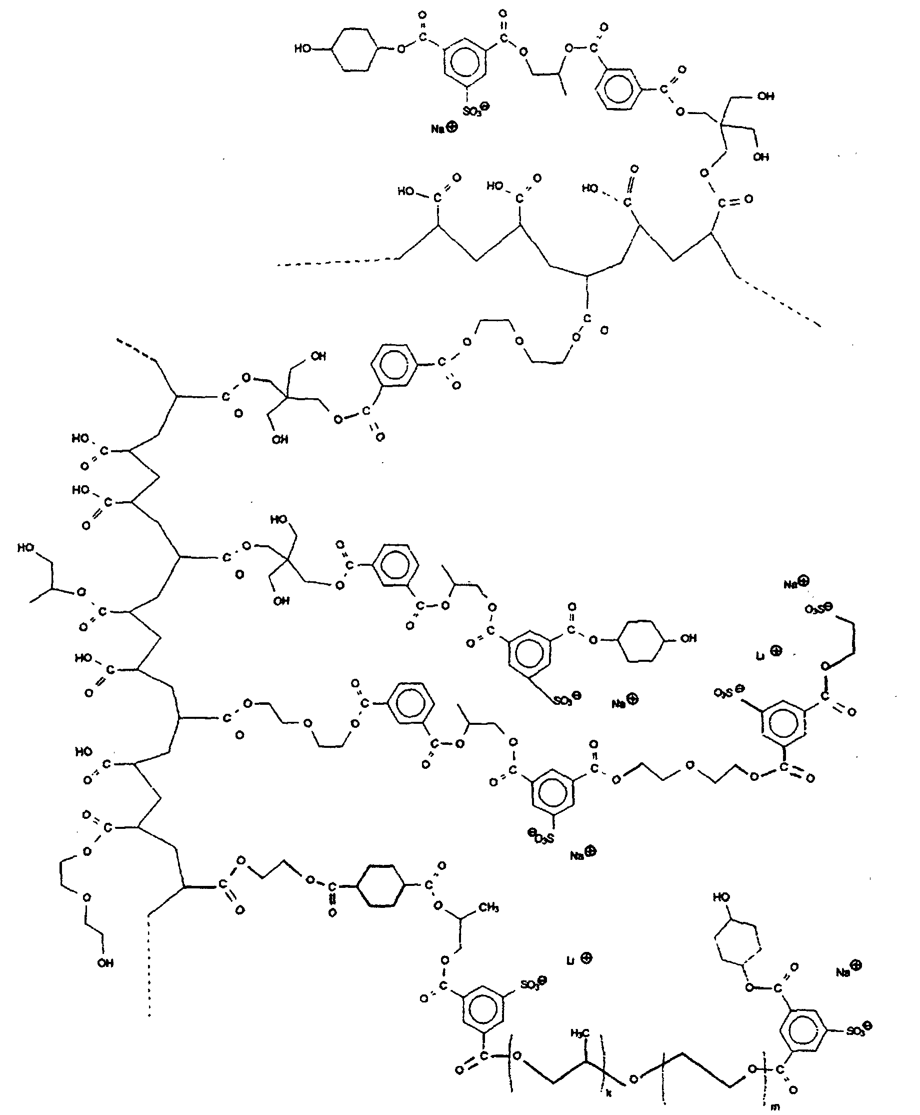

Die erfindungsgemäß einzusetzenden, sulfonhaltigen Polyester sind farblose bis gelbliche, geruchsneutrale Feststoffe. Sie sind in Wasser und Alkoholen gut löslich. Sie können vorteilhaft in kosmetische Zubereitungen zur Festigung der Haare eingearbeitet werden.

Die Herstellung erfindungsgemäßer Kammpolymere erfolgt vorteilhaft, indem ein oder mehrere mehrfunktionelle Alkohole mit einer sulfonsäuregruppenhaltigen, mindestens zwei Carboxylgruppen enthaltenden Substanz, beispielsweise 5-Sulfoisophthalsäuredimethylester-Na-Salz, gegebenenfalls einer weiteren mindestens zwei Carboxylgruppen enthaltenden Substanz und einem Polymer mit einer oder mehreren Polycarbonsäuren, beispielsweise Polyacrylsäure oder Polymethacrylsäure zusammengegeben, erhitzt und den üblichen Aufbereitungsschritten unterworfen werden.

In einer besonderen Ausführungsform der vorliegenden Erfindung werden die erfindungsgemäßen wasserlöslichen und/oder wasserdispergierbaren Kammpolymeren, bestehend aus einer Polyacrylsäure-enthaltenden Polymerhauptkette und sulfongruppenhaltigen Polyester-Seitenarmen daher in kosmetische, insbesondere haarkosmetische Zubereitungen eingearbeitet.

Zur Anwendung werden die erfindungsgemäßen kosmetischen und dermatologischen Zubereitungen in der für Kosmetika üblichen Weise auf die Haare in ausreichender Menge aufgebracht.

Die erfindungsgemäßen kosmetischen und dermatologischen Zubereitungen können kosmetische Hilfsstoffe enthalten, wie sie üblicherweise in solchen Zubereitungen verwendet werden, z.B. Konservierungsmittel, Bakterizide, Parfüme, Substanzen zum Verhindern des Schäumens, Farbstoffe, Pigmente, die eine färbende Wirkung haben, Verdickungsmittel, oberflächenaktive Substanzen, Emulgatoren, weichmachende, anfeuchtende und/oder feuchthaltende Substanzen, Fette, Öle, Wachse oder andere übliche Bestandteile einer kosmetischen oder dermatologischen Formulierung wie Alkohole, Polyole, Polymere, Schaumstabilisatoren, Elektrolyte, organische Lösungsmittel oder Siliconderivate.

In kosmetischen Zubereitungen zur Festigung der Haare, wie z.B. Haarsprays, Haarlacke, Schaumfestiger, Flüssigfestiger, Stylinggele usw., können die erfindungsgemäß einzusetzenden Kammpolymere vorzugsweise in Konzentrationen von 0.5 bis 30 Gewichtsprozent eingesetzt werden.

Die erfindungsgemäßen Zusammensetzungen zur Festigung der Haare können als Haarsprays oder Schaumaerosole vorliegen und die dafür üblichen und dem Stand der Technik entsprechenden Zusätze enthalten, sofern eine entsprechende Kompatibilität vorliegt. Dies sind beispielsweise weitere Lösungsmittel wie niedere Polyalkohole und deren toxikologisch verträglichen Ether und Ester, Weichmacher, leicht- und schwerflüchtige Silicone, leicht- und schwerflüchtige verzweigte bzw. unverzweigte Kohlenwasserstoffe, Emulgatoren, Antioxidantien, Wachse, Stabilisatoren, pH-Wert-Regulatoren, Farbstoffe, Konsistenzgeber, Antistatika, UV-Absorber, Parfums, usw.

Soll die erfindungsgemäße Zusammensetzung als Haarspray oder Schaumaerosol verwendet werden, so wird in der Regel ein Treibmittel zugesetzt. Übliche Treibmittel sind niedere Alkane, beispielsweise Propan, Butan oder Isobutan, Dimethylether, Stickstoff, Stickstoffdioxid oder Kohlendioxid oder Gemische aus diesen Substanzen.

Bei Verwendung in mechanischen Sprüh- oder Schaumvorrichtungen, beispielsweise Sprühpumpen oder manuellen Schaumpumpen bzw. Squeeze-systemen, kann das Treibmittel in der Regel entfallen.

Die wäßrigen erfindungsgemäßen Zubereitungen enthalten gegebenenfalls vorteilhaft Alkohole, Diole oder Polyole niedriger C-Zahl, sowie deren Ether, vorzugsweise Ethanol, Isopropanol, Propylenglykol, Glycerin, Ethylenglykol, Ethylenglykolmonoethyl- oder -monobutylether, Propylenglykolmonomethyl, -monoethyl- oder -monobutylether, Diethylenglykolmonomethyl- oder -monoethylether und analoge Produkte, ferner Alkohole niedriger C-Zahl, z.B. Ethanol, Isopropanol, 1,2-Propandiol, Glycerin sowie insbesondere ein oder mehrere Verdickungsmittel, welches oder welche vorteilhaft gewählt werden können aus der Gruppe Siliciumdioxid, Aluminiumsilikate, Polysaccharide bzw. deren Derivate, z.B. Hyaluronsäure, Xanthangummi, Hydroxypropylmethylcellulose, besonders vorteilhaft aus der Gruppe der Polyacrylate, bevorzugt ein Polyacrylat aus der Gruppe der sogenannten Carbopole, beispielsweise Carbopole der Typen 980, 981, 1382, 2984, 5984, jeweils einzeln oder in Kombination.

Im technischen Sinne werden unter Gelen verstanden: Relativ formbeständige, leicht verformbare disperse Systeme aus zumindest zwei Komponenten, welche in der Regel aus einem - meist festen - kolloid zerteilten Stoff aus langkettigen Molekülgruppierungen (z.B. Gelatine, Kieselsäure, Polysaccharide) als Gerüstbildner und einem flüssigen Dispersionsmittel (z.B. Wasser) bestehen. Der kolloidal zerteilte Stoff wird oft als Verdickungs- oder Geliermittel bezeichnet. Er bildet ein räumliches Netzwerk im Dispersionsmittel, wobei einzelne kolloidal vorliegende Partikel über elektrostatische Wechselwirkung miteinander mehr oder weniger fest verknüpft sein können. Das Dispersionsmittel, welches das Netzwerk umgibt, zeichnet sich durch elektrostatische Affinität zum Geliermittel aus, d.h., ein vorwiegend polares (insbesondere: hydrophiles) Geliermittel geliert vorzugsweise ein polares Dispersionsmittel (insbesondere: Wasser), wohingegen ein vorwiegend unpolares Geliermittel vorzugsweise unpolare Dispersionsmittel geliert.

Starke elektrostatische Wechselwirkungen, welche beispielsweise in Wasserstoffbrükkenbindungen zwischen Geliermittel und Dispersionsmittel, aber auch zwischen Dispersionsmittelmolekülen untereinander verwirklicht sind, können zu starker Vernetzung auch des Dispersionsmittels führen. Hydrogele können zu fast 100 % aus Wasser bestehen (neben beispielsweise ca. 0,2 - 1,0 % eines Geliermittels) und dabei durchaus feste Konsistenz besitzen. Der Wasseranteil liegt dabei in eisähnlichen Strukturelementen vor, so daß Gele daher ihrer Namensherkunft [aus lat. "gelatum" = "Gefrorenes" über den alchimistischen Ausdruck "gelatina" (16. Jhdt.) für nhdt. "Gelatine"] durchaus gerecht werden.

Gele gemäß der Erfindung enthalten üblicherweise Alkohole niedriger C-Zahl, z.B. Ethanol, Isopropanol, 1,2-Propandiol, Glycerin und Wasser in Gegenwart eines Verdickungsmittels, das bei ölig-alkoholischen Gelen vorzugsweise Siliciumdioxid oder ein Aluminiumsilikat, bei wäßrig-alkoholischen oder alkoholischen Gelen vorzugsweise ein Polyacrylat ist.

Bei kosmetischen und dermatologischen Zubereitungen gemäß der Erfindung beispielsweise kann es sich beispielsweise auch um Shampoonierungsmittel, Zubereitungen zum Fönen oder Einlegen der Haare, Zubereitungen zum Färben, um eine Frisieroder Behandlungslotion handeln.

Erfindungsgemäße Zubereitungen können sich gegebenenfalls vorteilhaft durch einen Gehalt an Tensiden auszeichnen. Tenside sind amphiphile Stoffe, die organische, unpolare Substanzen in Wasser lösen können. Sie sorgen, bedingt durch ihren spezifischen Molekülaufbau mit mindestens einem hydrophilen und einem hydrophoben Molekülteil, für eine Herabsetzung der Oberflächenspannung des Wassers, die Benetzung der Haut, die Erleichterung der Schmutzentfernung und -lösung, ein leichtes Abspülen und - je nach Wunsch - für Schaumregulierung.

Bei den hydrophilen Anteilen eines Tensidmoleküls handelt es sich meist um polare funktionelle Gruppen, beispielsweise -COO⁻, -OSO₃²⁻, -SO₃⁻, während die hydrophoben Teile in der Regel unpolare Kohlenwasserstoffreste darstellen. Tenside werden im allgemeinen nach Art und Ladung des hydrophilen Molekülteils klassifiziert. Hierbei können vier Gruppen unterschieden werden:
- anionische Tenside,
- kationische Tenside,
- amphotere Tenside und
- nichtionische Tenside.

Anionische Tenside weisen als funktionelle Gruppen in der Regel Carboxylat-, Sulfatoder Sulfonatgruppen auf. In wäßriger Lösung bilden sie im sauren oder neutralen Milieu negativ geladene organische lonen. Kationische Tenside sind beinahe ausschließlich durch das Vorhandensein einer quartemären Ammoniumgruppe gekennzeichnet In wäßriger Lösung bilden sie im sauren oder neutralen Milieu positiv geladene organische Ionen. Amphotere Tenside enthalten sowohl anionische als auch kationische Gruppen und verhalten sich demnach in wäßriger Lösung je nach pH-Wert wie anionische oder kationische Tenside. Im stark sauren Milieu besitzen sie eine positive und im alkalischen Milieu eine negative Ladung. Im neutralen pH-Bereich hingegen sind sie zwitterionisch, wie das folgende Beispiel verdeutlichen soll:
RNH₂⁺CH₂CH₂COOH X⁻ (bei pH=2) X⁻ = beliebiges Anion, z.B. Cl⁻
RNH₂⁺CH₂CH₂COO⁻ (bei pH=7)
RNHCH₂CH₂COO⁻ B⁺ (bei pH=12) B⁺ = beliebiges Kation, z.B. Na⁺

Typisch für nicht-ionische Tenside sind Polyether-Ketten. Nicht-ionische Tenside bilden in wäßrigem Medium keine Ionen.

### A. Anionische Tenside

Vorteilhaft zu verwendende anionische Tenside sind
Acylaminosäuren (und deren Salze), wie
1. Acylglutamate, beispielsweise Natriumacylglutamat, Di-TEA-palmitoylaspartat und Natrium Caprylic/ Capric Glutamat,
2. Acylpeptide, beispielsweise Palmitoyl-hydrolysiertes Milchprotein, Natrium Cocoyl-hydrolysiertes Soja Protein und Natrium-/ Kalium Cocoyl-hydrolysiertes Kollagen,
3. Sarcosinate, beispielsweise Myristoyl Sarcosin, TEA-lauroyl Sarcosinat, Natriumlauroylsarcosinat und Natriumcocoylsarkosinat,
4. Taurate, beispielsweise Natriumlauroyltaurat und Natriummethylcocoyltaurat,
5. AcylLactylate, lauroyllactylat, Caproyllactylat
6. Alaninate
Carbonsäuren und Derivate, wie
1. Carbonsäuren, beispielsweise Laurinsäure, Aluminiumstearat, Magnesiumalkanolat und Zinkundecylenat,
2. Ester-Carbonsäuren, beispielsweise Calciumstearoyllactylat, Laureth-6 Citrat und Natrium PEG-4 Lauramidcarboxylat,
3. Ether-Carbonsäuren, beispielsweise Natriumlaureth-13 Carboxylat und Natrium PEG-6 Cocamide Carboxylat,

Phosphorsäureester und Salze, wie beispielsweise DEA-Oleth-10-Phosphat und Dilaureth-4 Phosphat,

Sulfonsäuren und Salze, wie
1. Acyl-isethionate, z.B. Natrium-/ Ammoniumcocoyl-isethionat,
2. Alkylarylsulfonate,
3. Alkylsulfonate, beispielsweise Natriumcocosmonoglyceridsulfat, Natrium C₁₂₋₁₄ Olefin-sulfonat, Natriumlaurylsulfoacetat und Magnesium PEG-3 Cocamidsulfat,
4. Sulfosuccinate, beispielsweise Dioctylnatriumsulfosuccinat, Dinatriumlaurethsulfosuccinat, Dinatriumlaurylsulfosuccinat und Dinatriumundecylenamido MEA-Sulfosuccinat
sowie

Schwefelsäureester, wie
1. Alkylethersulfat, beispielsweise Natrium-, Ammonium-, Magnesium-, MIPA-, TIPA-Laurethsulfat, Natriummyrethsulfat und Natrium C₁₂₋₁₃ Parethsulfat,
2. Alkylsulfate, beispielsweise Natrium-, Ammonium- und TEA- Laurylsulfat.

### B. Kationische Tenside

Gegebenenfalls vorteilhaft zu verwendende kationische Tenside sind
1. Alkylamine,
2. Alkylimidazole,
3. Ethoxylierte Amine und
4. Quatemäre Tenside.
5. Esterquats

Quatemäre Tenside enthalten mindestens ein N-Atom, das mit 4 Alkyl- oder Arylgruppen kovalent verbunden ist. Dies führt, unabhängig vom pH Wert, zu einer positiven Ladung. Vorteilhaft sind, Alkylbetain, Alkylamidopropylbetain und Alkyl-amidopropylhydroxysulfain. Die erfindungsgemäß verwendeten kationischen Tenside können ferner bevorzugt gewählt werden aus der Gruppe der quaternären Ammoniumverbindungen, insbesondere Benzyltrialkylammoniumchloride oder -bromide, wie beispielsweise Benzyldimethylstearylammoniumchlorid, ferner Alkyltrialkylammoniumsalze, beispielsweise Cetyltrimethylammoniumchlorid oder -bromid, Alkyldimethylhydroxyethylammoniumchloride oder -bromide, Dialkyldimethylammoniumchloride oder -bromide, Alkylamidethyltrimethylammoniumethersulfate, Alkylpyridiniumsalze, beispielsweise Lauryl- oder Cetylpyrimidiniumchlorid, Imidazolinderivate und Verbindungen mit kationischem Charakter wie Aminoxide, beispielsweise Alkyldimethylaminoxide oder Alkylaminoethyldimethylaminoxide. Vorteilhaft sind insbesondere Cetyltrimethylammoniumsalze zu verwenden.

### C. Amphotere Tenside

Vorteilhaft zu verwendende amphotere Tenside sind
1. Acyl-/dialkylethylendiamin, beispielsweise Natriumacylamphoacetat, Dinatriumacylamphodipropionat, Dinatriumalkylamphodiacetat, Natriumacylamphohydroxypropylsulfonat, Dinatriumacylamphodiacetat und Natriumacylamphopropionat,
2. N-Alkylaminosäuren, beispielsweise Aminopropylalkylglutamid, Alkylaminopropionsäure, Natriumalkylimidodipropionat und Lauroamphocarboxyglycinat.

### D. Nicht-ionische Tenside

Vorteilhaft zu verwendende nicht-ionische Tenside sind
1. Alkohole,
2. Alkanolamide, wie Cocamide MEA/ DEA/ MIPA,
3. Aminoxide, wie Cocoamidopropylaminoxid,
4. Ester, die durch Veresterung von Carbonsäuren mit Ethylenoxid, Glycerin, Sorbitan oder anderen Alkoholen entstehen,
5. Ether, beispielsweise ethoxylierte/propoxylierte Alkohole, ethoxylierte/ propoxylierte Ester, ethoxylierte/ propoxylierte Glycerinester, ethoxylierte/ propoxylierte Cholesterine, ethoxylierte/ propoxylierte Triglyceridester, ethoxyliertes propoxyliertes Lanolin, ethoxylierte/ propoxylierte Polysiloxane, propoxylierte POE-Ether und Alkylpolyglycoside wie Laurylglucosid, Decylglycosid und Cocoglycosid.
6. Sucroseester, -Ether
7 Polyglycerinester, Diglycerinester, Monoglycerinester
8. Methylglucosester, Ester von Hydroxysäuren

Vorteilhaft ist ferner die Verwendung einer Kombination von anionischen und/oder amphoteren Tensiden mit einem oder mehreren nicht-ionischen Tensiden.

In der Regel ist im Sinne der vorliegenden Erfindung die Verwendung von anionischen, amphoteren und/oder nicht-ionischen Tensiden gegenüber der Verwendung von kationischen Tensiden bevorzugt.

Die kosmetischen und dermatologischen Zubereitungen enthalten gegebenenfalls Wirkstoffe und Hilfsstoffe, wie sie üblicherweise für diesen Typ von Zubereitungen zur Haarpflege und Haarbehandlung verwendet werden. Als Hilfsstoffe dienen Konservierungsmittel, oberflächenaktive Substanzen, Substanzen zum Verhindern des Schäumens, Verdickungsmittel, Emulgatoren, Fette, Öle, Wachse, organische Lösungsmittel, Bakterizide, Parfüme, Farbstoffe oder Pigmente, deren Aufgabe es ist, die Haare oder die kosmetische oder dermatologische Zubereitung selbst zu färben, Elektroyte, Substanzen gegen das Fetten der Haare.

Unter Elektrolyten im Sinne der vorliegenden Erfindung sind wasserlösliche Alkali-, Ammonium-, Erdalkali- (unter Einbeziehung des Magnesiums) und Zinksalze anorganischer Anionen und beliebige Gemische aus solchen Salzen zu verstehen, wobei gewährleistet sein muß, daß sich diese Salze durch pharmazeutische oder kosmetische Unbedenklichkeit auszeichnen.

Die erfindungsgemäßen Anionen werden bevorzugt gewählt aus der Gruppe der Chloride, der Sulfate und Hydrogensulfate, der Phosphate, Hydrogenphosphate und der linearen und cyclischen Oligophosphate sowie der Carbonate und Hydrogencarbonate.

Kosmetische Zubereitungen, die ein Shampoonierungsmittel darstellen, enthalten vorzugsweise mindestens eine anionische, nicht-ionische oder amphotere oberflächenaktive Substanz, oder auch Gemische aus solchen Substanzen im wäßrigen Medium und Hilfsmittel, wie sie üblicherweise dafür verwendet werden. Die oberflächenaktive Substanz bzw. die Gemische aus diesen Substanzen können in einer Konzentration zwischen 1 Gew.-% und 50 Gew.-% in dem Shampoonierungsmittel vorliegen.

Eine kosmetische Zubereitung in Form einer Lotion, die nicht ausgespült wird, insbesondere eine Lotion zum Einlegen der Haare, eine Lotion, die beim Fönen der Haare verwendet wird, eine Frisier- und Behandlungslotion, stellt im allgemeinen eine wäßrige, alkoholische oder wäßrig-alkoholische Lösung dar und enthält die erfindungsgemäßen Kammpolymere.

Die erfindungsgemäßen Zusammensetzungen enthalten gegebenenfalls die in der Kosmetik üblichen Zusatzstoffe, beispielsweise Parfüm, Verdicker, Farbstoffe, Desodorantien, antimikrobielle Stoffe, rückfettende Agentien, Komplexierungs- und Sequestrierungsagentien, Perlglanzagentien, Pflanzenextrakte, Vitamine, Wirkstoffe und dergleichen. Die nachfolgenden Beispiele sollen die vorliegende Erfindung verdeutlichen, ohne sie einzuschränken. Alle Mengenangaben, Anteile und Prozentanteile sind, soweit nicht anders angegeben, auf das Gewicht und die Gesamtmenge bzw. auf das Gesamtgewicht der Zubereitungen bezogen.

### (A) Herstellunasbeispiele

### Herstellungsbeispiel 1

| Edukt | Masse (g) |
|---|---|
| Isophthalsäure | 265,81 |
| 5-Sulfoisophthalsäuremethylester, Na-Salz | 100,52 |
| 5-Sulfoisophthalsäure, Li-Salz | 22,92 |
| Isethionsäure, Na-Salz | 10,96 |
| Polyacrylsäure (Mₙ =2.000 g/mol) | 3,00 |
| Natriumcarbonat | 0,60 |
| Titantetraisopropylat | 0,60 |
| 1,2-Propandiol | 195,40 |
| Diethylenglycol | 166,95 |

### Herstellweise:

In einem 2 I Vierhalskolben mit KPG-Rührer, Innenthermometer, Gaseinleitungsrohr und Destillationsbrücke werden 1,2-Propandiol, Diethylenglykol, 5-Sulfoisophthalsäuremethylester-Na-Salz, 5-Sulfoisophthalsäure-Li-Salz, Natriumcarbonat und Titantetraisopropylat vorgelegt, und bei 180 °C 5 Stunden lang umgeestert. Das dabei entstehende Methanol und Reaktionswasser werden bei Normaldruck abdestilliert. Anschließend werden das Natriumsalz der Isethionsäure, Isophthalsäure und Polyacrylsäure zur Reaktionsmischung hinzugegeben, hernach gut mit N₂ inertisiert. Die Reaktionsmischung wird dann langsam auf 220 °C aufgeheizt und solange bei dieser Temperatur gehalten, bis kein weiteres Kondensat überdestilliert. In einem Zeitraume von drei Stunden wird dann, von Normaldruck ausgehend, der Innendruck des Reaktionsgefäßes auf Ölpumpenvakuum abgesenkt Nach weiteren 60 Minuten wird mit N₂ belüftet und die heiße Polymerschmelze ausgetragen.

### Herstellungsbeispiel 2

| Edukt | Mol (mmol) |
|---|---|
| 1,3-Cyclohexandicarbonsäure | 132,80 |
| Terephthalsäuredimethylester | 95,12 |
| 5-Sulfoisophthalsäuremethylester, Na-Salz | 75,20 |
| 5-Sulfoisophthalsäure, Li-Salz | 75,20 |
| Polyacrylsäure (Mₙ =5.000 g/mol) | 7,00 |
| Natriumcarbonat | 0,60 |
| Titantetraisopropylat | 0,80 |
| 1,2-Propandiol | 195,40 |
| Diethylenglycol | 166,95 |

### Herstellweise:

In einem 2 I Vierhalskolben mit KPG-Rührer, Innenthermometer, Gaseinleitungsrohr und Destillationsbrücke werden 1,2-Propandiol, Diethylenglykol, 5-Sulfoisophthalsäuremethylester-Na-Salz, 5-Sulfoisophthalsäure-Li-Salz, Natriumcarbonat und Titantetraisopropylat vorgelegt, und bei 180 °C 5 Stunden lang umgeestert. Das dabei entstehende Methanol und Reaktionswasser werden bei Normaldruck abdestilliert. Anschließend werden das Natriumsalz der Isethionsäure, 1,3-Cyclohexandicarbonsäure und Polyacrylsäure zur Reaktionsmischung hinzugegeben, hernach gut mit N₂ inertisiert. Die Reaktionsmischung wird dann langsam auf 220 °C aufgeheizt und solange bei dieser Temperatur gehalten, bis kein weiteres Kondensat überdestilliert. In einem Zeitraume von drei Stunden wird dann, von Normaldruck ausgehend, der Innendruck des Reaktionsgefäßes auf Ölpumpenvakuum abgesenkt. Nach weiteren 60 Minuten wird mit N₂ belüftet und die heiße Polymerschmelze ausgetragen.

### Herstellungsbeispiel 3

| Edukt | Masse (g) |
|---|---|
| 2,6-Naphthalindicarbonsäure | 172,95 |
| 5-Sulfoisophthalsäuremethylester, Na-Salz | 55,52 |
| 5-Sulfoisophthalsäure, Li-Salz | 97,13 |
| Isophthalsäure | 55,00 |
| Poly[acrylsäure-co-methacrylsäuremethylester Mₙ = 5.000 g/mol) * | 3,00 |
| Natriumcarbonat | 0,60 |
| Titantetraisopropylat | 0,60 |
| 1,2-Propandiol | 188,40 |
| Diethylenglycol | 155,12 |

### Herstellweise:

In einem 2 I Vierhalskolben mit KPG-Rührer, Innenthermometer, Gaseinleitungsrohr und Destillationsbrücke werden 1,2-Propandiol, Diethylenglykol, 5-Sulfoisophthalsäuremethylester-Na-Salz, 5-Sulfoisophthalsäure-Li-Salz, Natriumcarbonat und Titantetraisopropylat vorgelegt, und bei 180 °C 5 Stunden lang umgeestert. Das dabei entstehende Methanol und Reaktionswasser werden bei Normaldruck abdestilliert. Anschließend werden 2,6-Naphthalindicarbonsäure, Isophthalsäure und Poly[acrylsäure-comethacrylsäuremethylester zur Reaktionsmischung hinzugegeben, hernach gut mit N₂ inertisiert. Die Reaktionsmischung wird dann langsam auf 220 °C aufgeheizt und solange bei dieser Temperatur gehalten, bis kein weiteres Kondensat überdestilliert. In einem Zeitraume von drei Stunden wird dann, von Normaldruck ausgehend, der Innendruck des Reaktionsgefäßes auf Ölpumpenvakuum abgesenkt. Nach weiteren 75 Minuten wird mit N₂ belüftet und die heiße Polymerschmelze ausgetragen.

### Herstellungsbeispiel 4

| Edukt | Mol (mmol) |
|---|---|
| Adipinsäure | 172,95 |
| 5-Sulfoisophthalsäuremethylester, Na-Salz | 55,52 |
| 5-Sulfoisophthalsäure, Li-Salz | 97,13 |
| Isophthalsäure | 55,00 |
| Polyacrylsäure (Nₙ = 5.000 g/mol) | 3,00 |
| Natriumcarbonat | 0,60 |
| Titantetraisopropylat | 0,60 |
| Diethylenglycol | 400,00 |

### Herstellweise:

In einem 2 I Vierhalskolben mit KPG-Rührer, Innenthermometer, Gaseinleitungsrohr und Destillationsbrücke werden Diethylenglykol, 5-Sulfoisophthalsäuremethylester-Na-Salz, 5-Sulfoisophthalsäure-Li-Salz, Natriumcarbonat, Titantetraisopropylat, Isophthalsäure, Polyacrylsäure und Adipinsäure vorgelegt, und langsam auf 200 °C aufgeheizt. Das dabei entstehende Methanol und Reaktionswasser werden bei Normaldruck abdestilliert. Anschließend wird ausreichend mit N₂ inertisiert und die Reaktionsmischung langsam auf 250 °C aufgeheizt und solange bei dieser Temperatur gehalten, bis kein Kondensat mehr überdestillliert. In einem Zeitraume von drei Stunden wird dann, von Normaldruck ausgehend, der Innendruck des Reaktionsgefäßes auf Ölpumpenvakuum abgesenkt. Nach weiteren 75 Minuten wird mit N₂ belüftet und die heiße Polymerschmelze ausgetragen.

### Herstellungsbeispiel 5

| Edukt | Mol (mmol) |
|---|---|
| 1,4-Cyclohexandicarbonsäure | 172,95 |
| 5-Sulfoisophthalsäuremethylester, Na-Salz | 55,52 |
| 5-Sulfoisophthalsäure, Li-Salz | 97,13 |
| Isophthalsäure | 55,00 |
| Polyacrylsäure (Mₙ = 5.000 g/mol) | 3,00 |
| Cyclohexandimethanol | 150,12 |
| Natriumcarbonat | 0,60 |
| Titantetraisopropylat | 0,60 |
| Diethylenglycol | 400,00 |

### Herstellweise:

In einem 2 I Vierhalskolben mit KPG-Rührer, Innenthermometer, Gaseinleitungsrohr und Destillationsbrücke werden Cyclohexandimethanol, Diethylenglykol, 5-Sulfoisophthalsäuremethylester-Na-Salz, 5-Sulfoisophthalsäure-Li-Salz, Natriumcarbonat, Titantetraisopropylat, Isophthalsäure, Polyacrylsäure und 1,4-Cyclohexandicarbonsäure vorgelegt, und langsam auf 200 °C aufgeheizt. Das dabei entstehende Methanol und Reaktionswasser werden bei Normaldruck abdestilliert. Anschließend wird ausreichend mit N₂ inertisiert und die Reaktionsmischung langsam auf 250 °C aufgeheizt und solange bei dieser Temperatur gehalten, bis kein Kondensat mehr überdestillliert. In einem Zeitraume von drei Stunden wird dann, von Normaldruck ausgehend, der Innendruck des Reaktionsgefäßes auf Ölpumpenvakuum abgesenkt. Nach weiteren 75 Minuten wird mit N₂ belüftet und die heiße Polymerschmelze ausgetragen.

### Herstellungsbeispiel 6

| Edukt | Masse (g) |
|---|---|
| Pentaerythrit | 7,20 |
| 5-Sulfoisophthalsäuremethylester, Na-Salz | 15,02 |
| 5-Sulfoisophthalsäure, Li-Salz | 140,21 |
| Isophthalsäure | 55,00 |
| Isethionsäure, Li-Salz | 25,98 |
| Polyacrylsäure (Mₙ = 12.000 g/mol) | 3,00 |
| Natriumcarbonat | 0,60 |
| Titantetraisopropylat | 0,60 |
| 1,4-Cyclohexandimethanol | 144,21 |
| Diethylenglycol | 400,00 |

### Herstellweise:

In einem 2 I Vierhaiskolben mit KPG-Rührer, Innenthermometer, Gaseinleitungsrohr und Destillationsbrücke werden Diethylenglykol, 5-Sulfoisophthalsäuremethylester-Na-Salz, 5-Sulfoisophthalsäure-Li-Salz, Natriumcarbonat, Titantetraisopropylat, Isophthalsäure, und Pentaerythrit vorgelegt, und langsam auf 200 °C aufgeheizt Das dabei entstehende Methanol und Reaktionswasser werden bei Normaldruck abdestilliert. Danach werden die Polyacrylsäure und das Isethionsäure-Li-Salz zur Reaktionsmischung zugesetzt. Anschließend wird ausreichend mit N₂ inertisiert und die Reaktionsmischung langsam auf 250 °C aufgeheizt und solange bei dieser Temperatur gehalten, bis kein Kondensat mehr überdestillliert. In einem Zeitraume von drei Stunden wird dann, von Normaldruck ausgehend, der Innendruck des Reaktionsgefäßes auf Ölpumpenvakuum abgesenkt. Nach weiteren 75 Minuten wird mit N₂ belüftet und die heiße Polymerschmelze ausgetragen.

### (B) Rezepturbeispiele:

### Haarsprays: Beispiele 1 - 6

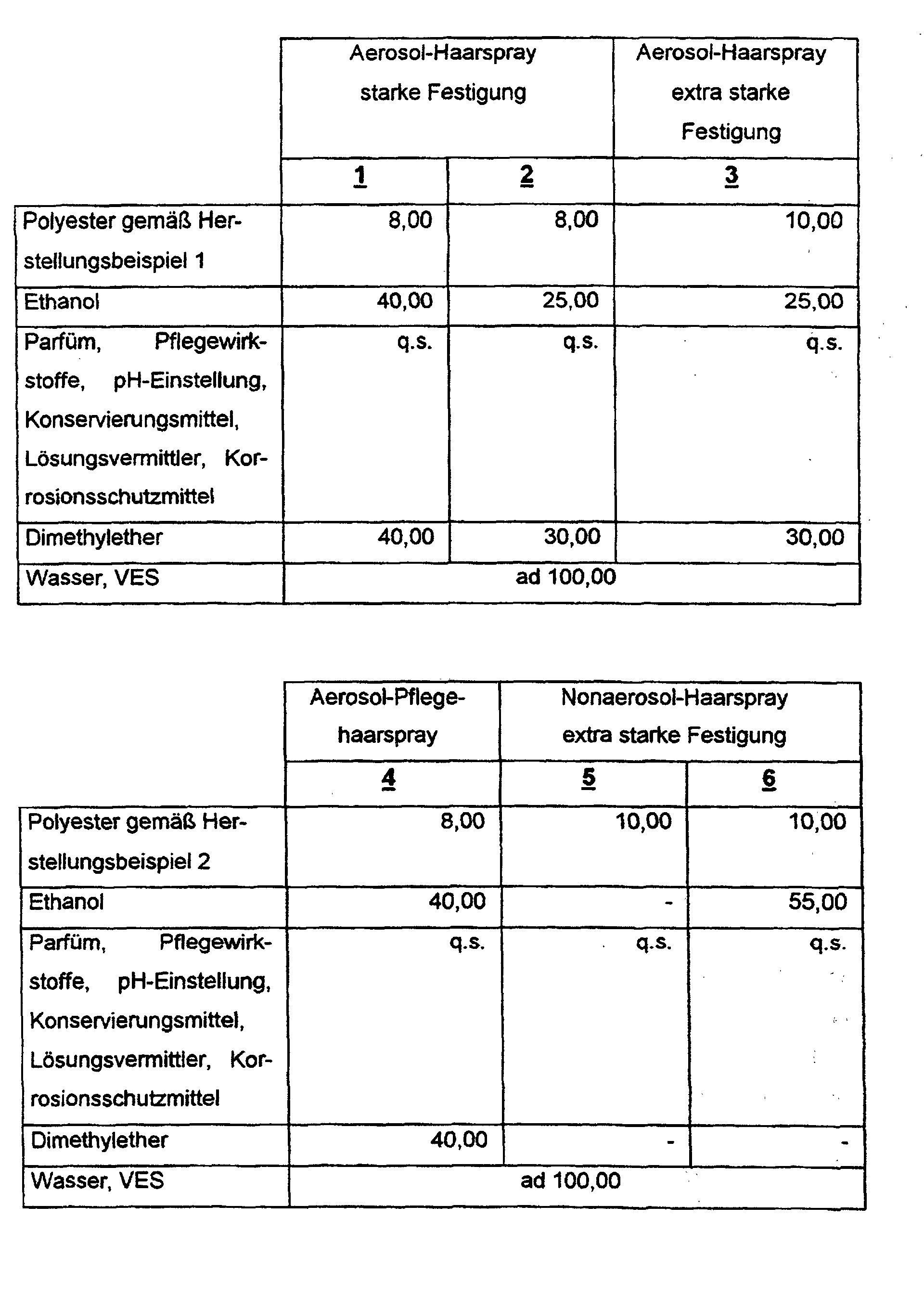

### Schaumfestiger: Beispiele 7 - 8

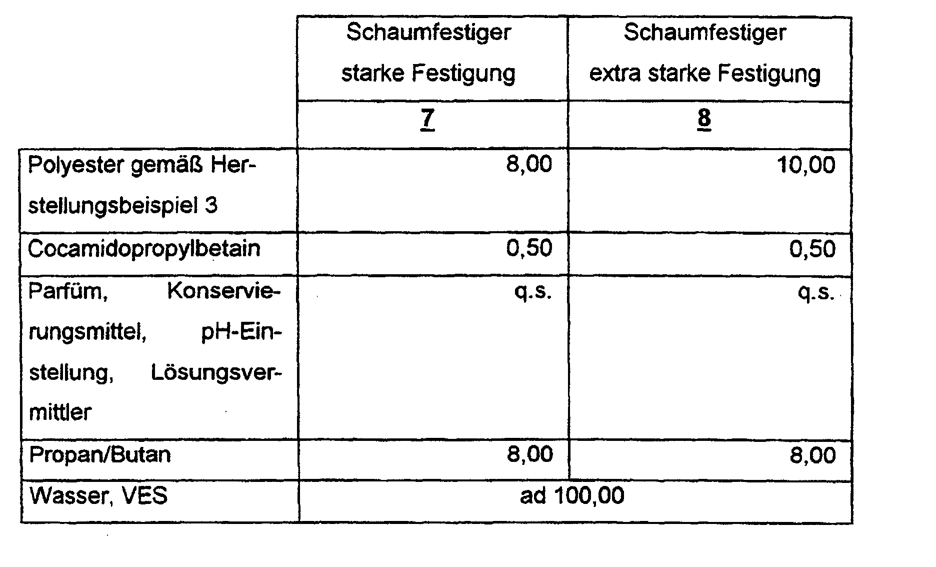

### Stylinggele: Beispiele 9 - 10

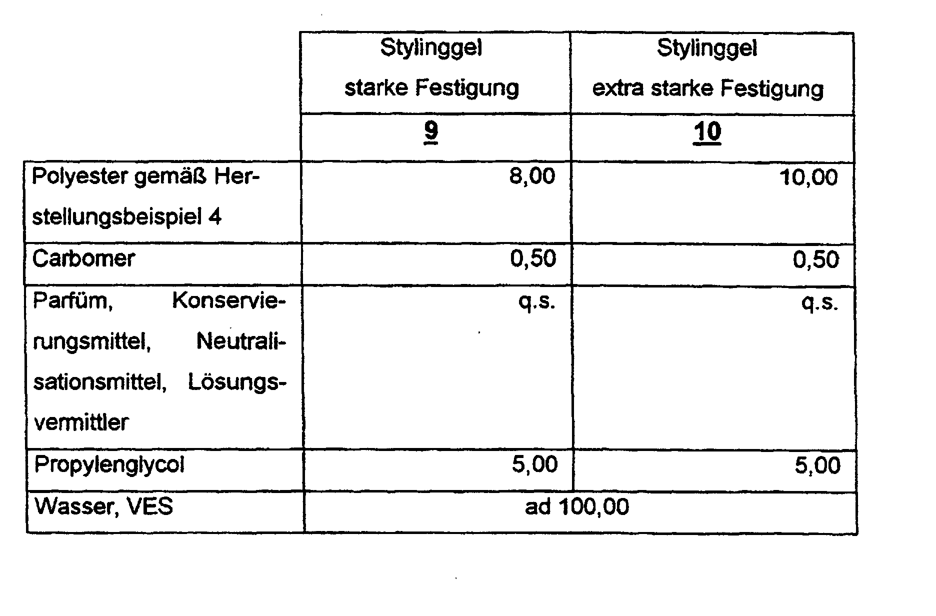

### Styling_Shampoos: Beispiel 11 - 12

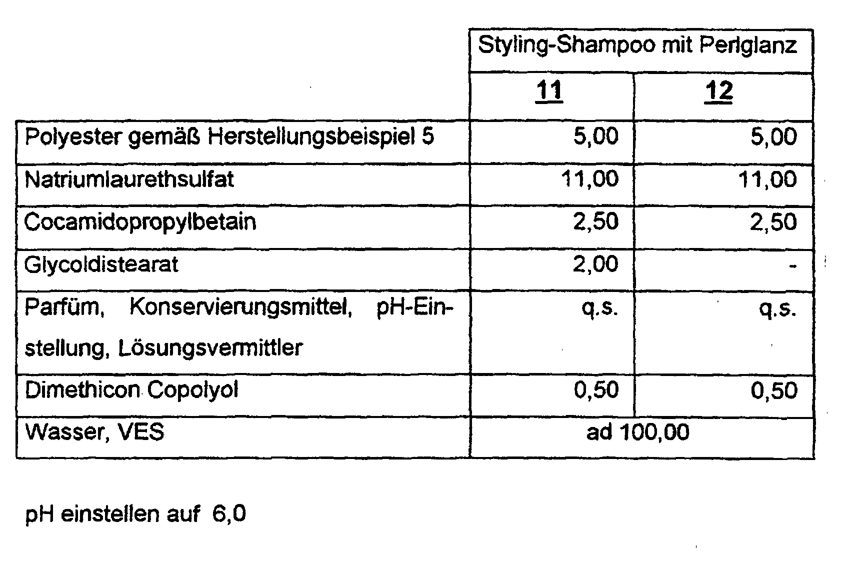

## Patentansprüche

1. Wasserlösliche und/oder wasserdispergierbare Kammpolymere, bestehend aus einer Polymerhauptkette und mit dieser Polymerhauptkette über Estergruppen verknüpften sulfongruppenhaltigen Polyesterseitenarmen, welche wenigstens teilweise durch Natrium und Lithiumgegenionen neutralisiert wurden, wobei das molare Verhältnis von Lithium zu Natrium zwischen 0,1 und 50, bevorzugt zwischen 0,5 und 25, liegt,

2. Kammpolymere nach Anspruch 1, **dadurch gekennzeichnet, daß** ihre polymere Hauptkette gewählt wird aus der Gruppe der polymeren aliphatischen, cycloaliphatischen oder aromatischen Polycarbonsäuren bzw. deren Derivaten wie beispielsweise Polyacrylsäure, Polymethacrylsäure und deren Ester (Ester der beiden Säuren mit aliphatischen, cycloaliphatischen oder aromatischen Alkoholen mit C₁ bis C₂₂), Maleinsäure, Maleinsäureanhydrid, Fumarsäure und Polynorbonensäure.

3. Kammpolymere nach Anspruch 1, **dadurch gekennzeichnet, daß** sie gewählt werden aus der Gruppe der Polyester folgender generischer Strukturformeln 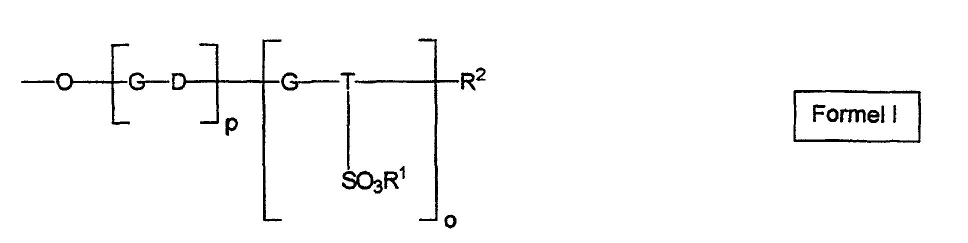 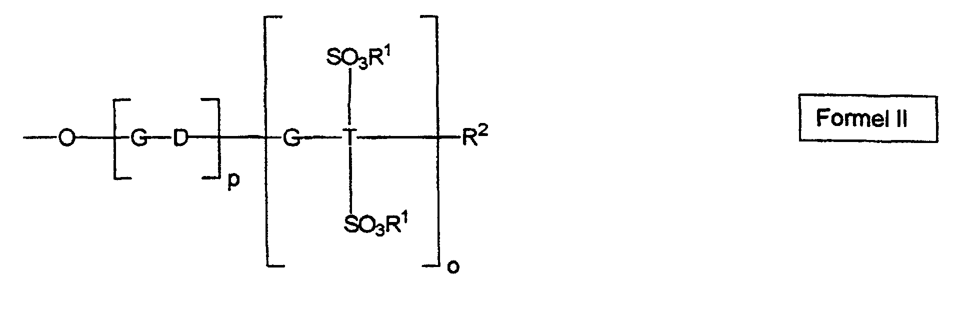 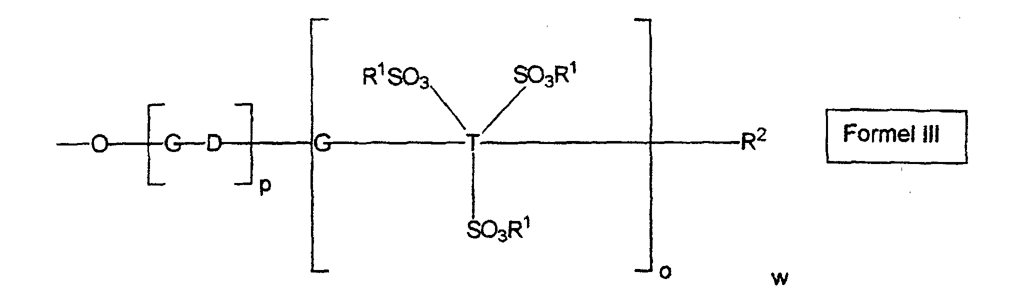 obei p und o so gewählt werden, daß mittlere Molekulargewichte der eingesetzten Hauptkettenbestandteile zwischen 200 und 2.000.000 g/mol liegen, wobei der Bereich von 2.000 -100.000 g/mol bevorzugt Verwendung findet,
die Polyester-Seitenketten gemäß Formel I - III vorteilhaft bestehen aus:
G : gewählt wird aus der Gruppe der mindestens zwei endständige Sauerstoffatome enthaltende aromatischen, aliphatischen oder cycloaliphatischen Organyleinheiten mit einer Kohlenstoffzahl von C₂ bis C₂₂ oder Abkömmlinge eines Polyglykols der Form HO-[R³-O)ₖ-[R⁴-O)ₘ-H, entsprechend einer Organyleinheit 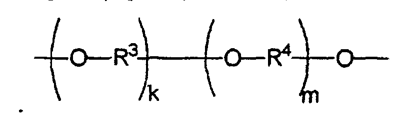
wobei die Reste R³ und R⁴ Alkylenreste darstellen mit einer Kohlenstoffzahl von C₂-C₂₂, wobei beide Reste nicht notwendigerweise verschieden sein müssen.
wobei für die Koeffizienten k und m gilt: k+m ≥ 1, wobei k und m ferner so gewählt werden können, daß die vorab bezeichneten mittleren Molekulargewichte der eingesetzten Hauptkettenbestandteile zuwege kommen.
D : einer mindestens zwei endständige Acylgruppen enthaltenden aromatischen, aliphatischen oder cycloaliphatischen Organyleinheit mit einer Kohlenstoffzahl von C₂ bis C₂₂, wobei auch Kombinationen aus mehreren verschiedenen Säurekomponenten im beanspruchten Zielmolekül enthalten sein können, beispielsweise eine Organyleinheit des Schemas 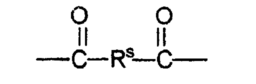
wobei R^{s} aromatische und lineare oder cyclische, gesättigte oder ungesättigte aliphatische bifunktionale Reste mit Kohlenstoffzahlen von C₂ bis C₂₂ darstellen kann.
T: eine Verbindung aus der Gruppe der mindestens zwei endständige Acylgruppen enthaltenden sulfonierten aromatischen, aliphatischen oder cycloaliphatischen Organylverbindungen
R¹: Lithium und/oder Natrium neben gegebenenfalls weiteren Gegenionen, z.B., Kalium, Magnesium, Calcium, Ammonium, Monoalkylammonium, Dialkylammonium, Trialkylammonium oder Tetraalkylammonium bedeuten kann, worin die Alkylpositionen der Amine unabhängig voneinander mit C₁ bis C₂₂-Alkylresten und 0 bis 3 Hydroxylgruppen besetzt sind.
R²: einen Molekülrest, gewählt aus den Gruppen der
- aromatischen, aliphatischen oder cycloaliphatischen Aminofunktionen: (-NH-R⁵, -NR⁵₂ wobei R⁵ einen Alkyl- oder Arylrest mit C₁ bis C₂₂ darstellen kann)
- aromatischen, aliphatischen oder cycloaliphatischen Monocarbonsäuregruppen: (-COOR⁶ wobei R⁶ ein Alkyl- oder Arylrest darstellt mit C₁ bis C₂₀₀)
- über Etherfunktionen verbrückten aromatischen, aliphatischen oder cycloaliphatischen Organylreste: (-O-R⁵)
- über Etherfunktionen verbrückenden Polyalkoxyverbindungen der Form
-O-[R⁷-O]_{q}-[R⁸-O]ᵣ-Y
Die Reste R⁷ und R⁸ stellen vorteilhaft Alkylreste dar mit einer Kohlenstoffzahl von C₂-C₂₂, wobei beide Reste nicht notwendigerweise verschieden sein müssen. Der Rest Y kann sowohl Wasserstoff als auch aliphatischer Natur mit C₁-C₂₂ sein. Für die Koeffizienten q und r gilt: q+r ≥ 1.
- über Etherfunktionen verbrückenden einfach oder mehrfach ethoxylierten sulfonierten Organylreste oder bevorzugt deren Alkali- oder Erdalkalisalze, wie beispielsweise vorteilhaft **gekennzeichnet durch** die generische Strukturformel
-(O-CH₂-CH₂)ₛ-SO₃R¹
mit s ≥ 1, und wobei s ferner so gewählt werden kann, daß die vorab bezeichneten mittleren Molekulargewichte der eingesetzten Hauptkettenbestandteile zuwege kommen.

4. Kammpolymere nach Anspruch 1, **dadurch gekennzeichnet, daß** ihre mittleren Molekulargewichte vorteilhaft zwischen 200 und 2.000.000 g/mol liegen, besonders vorteilhaft zwischen 200 und 100.000 g/mol liegen wobei der Bereich von 1.000 - 30.000 g/mol bevorzugt Verwendung findet, ganz besonders vorteilhaft von 5.000 - 15.000 g/mol.

5. Haarbehandlungsmittel mit einem wirksamen Gehalt an einem oder mehreren Kammpolymeren nach einem der Ansprüche 1 - 4.

## Claims

1. Water-soluble and/or water-dispersible comb polymers consisting of a polymer main chain and polyester side-arms which contain sulphone groups and are linked to said polymer main chain via ester groups, which side-arms have been at least partially neutralized by sodium and lithium counterions, where the molar ratio of lithium to sodium is between 0.1 and 50, preferably between 0.5 and 25.

2. Comb polymers according to Claim 1, **characterized in that** their polymeric main chain is chosen from the group of polymeric aliphatic, cycloaliphatic or aromatic polycarboxylic acids and derivatives thereof such as, for example, polyacrylic acid, polymethacrylic acid and esters thereof (esters of the two acids with aliphatic, cycloaliphatic or aromatic alcohols with C₁ to C₂₂), maleic acid, maleic anhydride, fumaric acid and polynorbornenic acid.

3. Comb polymers according to Claim 1, **characterized in that** they are chosen from the group of polyesters of the following generic structural formulae 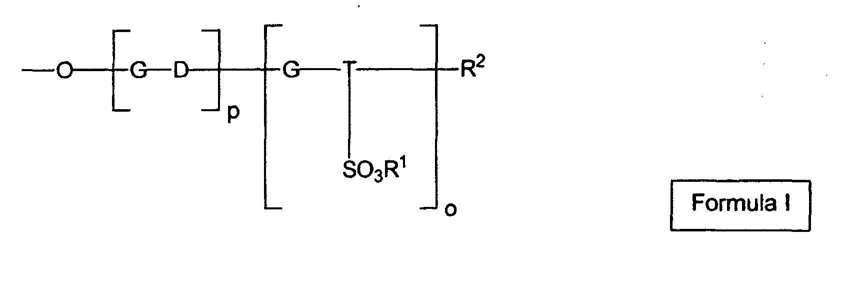 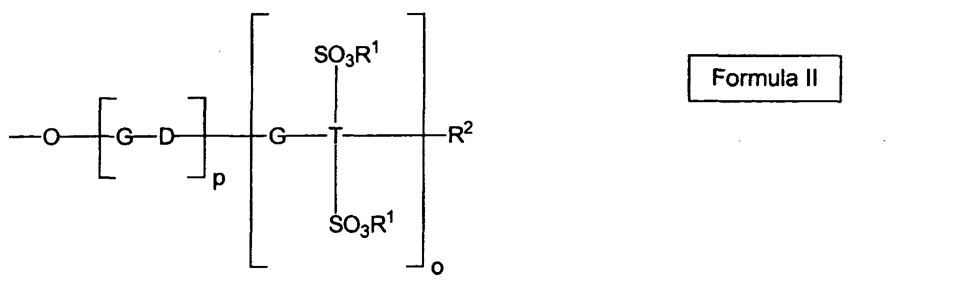 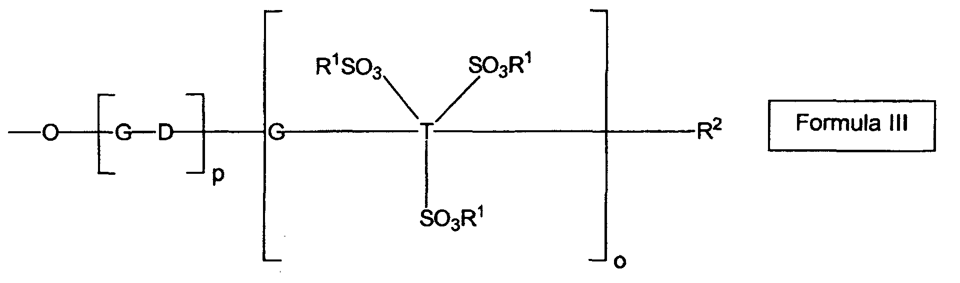 where p and o are chosen such that average molecular weights of the main chain constituents used are between 200 and 2,000,000 g/mol, where the range 2000 - 100,000 g/mol is preferably used,
the polyester side chains according to formula I - III advantageously consist of:
G : chosen from the group of aromatic, aliphatic or cyctoaliphatic organyl units having a carbon number of from C₂ to C₂₂ and containing at least two terminal oxygen atoms, or derivatives of a polyglycol of the form HO-[R³-O]ₖ-[R⁴-O]ₘ-H, corresponding to an organyl unit 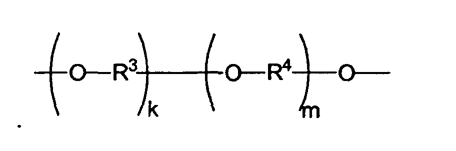
where the radicals R³ and R⁴ are alkylene radicals having a carbon number of from C₂-C₂₂, where the two radicals do not necessarily have to be different,
where the following applies for the coefficients k and m: k+m ≥ 1, where k and m can also be chosen such that the average molecular weights, referred to previously, of the main chain constituents used are achieved.
D : an aromatic, aliphatic or cycloaliphatic organyl unit having a carbon number of from C₂ to C₂₂ and containing at least two terminal acyl groups, where combinations of two or more different acid components may also be present in the claimed target molecule, for example an organyl unit of the scheme 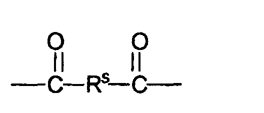
where R^{s} can be aromatic and linear or cyclic, saturated or unsaturated aliphatic bifunctional radicals having carbon numbers of from C₂ to C₂₂.
T : a compound from the group of the sulphonated aromatíc, aliphatic or cycloaliphaic organyl compounds containing at least two terminal acyl groups
R¹: can be lithium and/or sodium as well as, where appropriate, further counterions, e.g. potassium, magnesium, calcium, ammonium, monoalkylammonium, dialkylammonium, trialkylammonium or tetraalkylammonium, in which the alkyl positions of the amines are, independently of one another, occupied by C₁ to C₂₂-alkyl radicals and 0 to 3 hydroxyl groups.
R²: a molecular moiety chosen from the groups of
- aromatic, aliphatic or cycloaliphatic amino functions: ( -NH-R⁵, -NR⁵₂, where R⁵ can be an alkyl or aryl radical with C₁ to C₂₂)
- aromatic, aliphatic or cycloaliphatic monocarboxylic acid groups: (-COOR⁶, where R⁶ is an alkyl or aryl radical with C₁ to C₂₀₀)
- aromatic, aliphatic or cycloaliphatic organyl radicals bridged via ether functions: (-O-R⁵)
- polyalkoxy compounds bridging via ether functions and of the form
-O-[R⁷-O]_{q}-[R⁸-O]ᵣ-Y
The radicals R⁷ and R⁸ are advantageously alkyl radicals having a carbon number of from C₂ to C₂₂, where the two radicals do not necessarily have to be different. The radical Y can either be hydrogen or of an aliphatic nature with C₁-C₂₂. For the coefficients q and r the following applies: q+r ≥ 1.
- mono- or polyethoxylated sulphonated organyl radicals bridging via ether functions, or preferably alkali metal or alkaline earth metal salts thereof, such as, for example, advantageously **characterized by** the generic structural formula
-(O-CH₂-CH₂)ₛ-SO₃R¹
where s ≥ 1, and where s can also be chosen such that the average molecular weights, referred to previously, of the main chain constituents used are achieved.

4. Comb polymers according to Claim 1, **characterized in that** their average molecular weights are advantageously between 200 and 2,000,000 g/mol, particularly advantageously between 200 and 100,000 g/mol, the range 1000- 30,000 g/mol being preferably used, very particularly advantageously 5000 - 15,000 g/mol.

5. Hair-treatment compositions with an effective content of one or more comb polymers according to one of Claims 1 - 4.

## Revendications

1. Polymères en peigne hydrosolubles et/ou dispersables dans l'eau, constitués d'une chaîne principale polymère et de bras latéraux polyester renfermant des groupes sulfone liés à cette chaîne principale polymère par l'intermédiaire de groupes ester, lesquels bras latéraux ont été neutralisés au moins partiellement par des contre-ions sodium et lithium, le rapport molaire entre le lithium et le sodium étant compris entre 0,1 et 50, de préférence entre 0,5 et 25.

2. Polymères en peigne selon la revendication. 1, **caractérisés en ce que** leur chaîne principale polymère est choisie parmi le groupe constitué des acides polycarboxyliques polymères aliphatiques, cycloaliphatiques ou aromatiques et de leurs dérivés tels que, par exemple, l'acide polyacrylique, l'acide polyméthacrylique et leurs esters (esters des deux acides avec des alcools aliphatiques, cycloaliphatiques ou aromatiques en C₁ à C₂₂), l'acide maléique, l'anhydride maléique, l'acide fumarique et l'acide polynorbornénique.

3. Polymères en peigne selon la revendication 1, **caractérisés en ce qu'**ils sont choisis parmi le groupe constitué des polyesters de formules structurales génériques suivantes 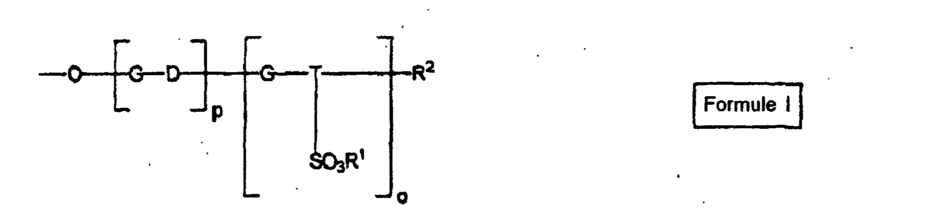 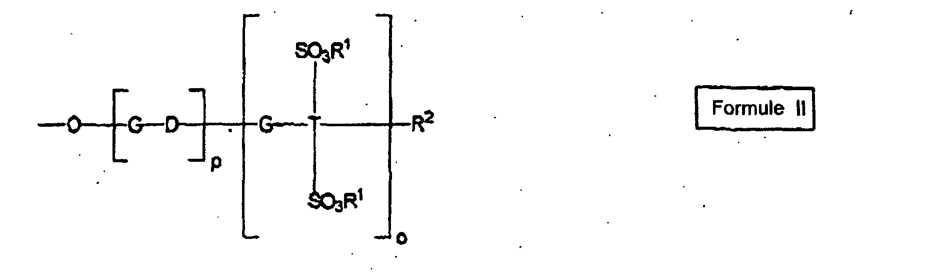 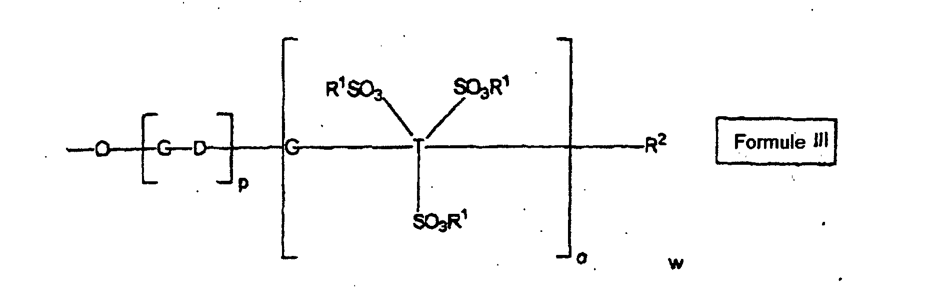 dans lesquelles p et o sont choisis de telle sorte que les poids moléculaires moyens des constituants de chaîne principale utilisés soient compris entre 200 et 2 000 000 g/mol, la plage de 2 000 à 100 000 g/mol étant de préférence utilisée,
les chaînes latérales polyester selon les formules I - III étant avantageusement constituées de :
G : choisi parmi le groupe constitué des motifs organyle aromatiques, aliphatiques ou cycloaliphatiques renfermant au moins deux atomes d'oxygène terminaux, avec un nombre de carbones de C₂ à C₂₂, ou des dérivés d'un polyglycol de formule HO-(R³-O)ₖ-[R⁴-O]ₘ-H correspondant à un motif organyle 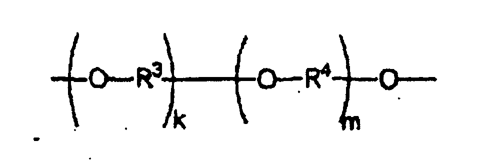
dans lequel les radicaux R³ et R⁴ représentent des radicaux alkylène ayant un nombre de carbones de C₂ à C₂₂, les deux radicaux n'étant pas nécessairement différents,
où pour les coefficients k et m : k + m ≥ 1, k et m pouvant en outre être choisis de telle sorte que les poids moléculaires moyens indiqués précédemment des constituants de chaîne principale utilisés soient obtenus
D : un motif organyle aromatique, aliphatique ou cycloaliphatique renfermant au moins deux groupes acyle terminaux, avec un nombre de carbones de C₂ à C₂₂, des combinaisons de plusieurs composants acides différents pouvant également être présents dans la molécule cible revendiquée, par exemple un motif organyle de formule 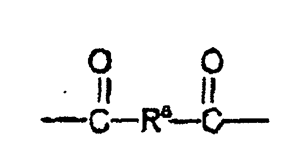 dans laquelle R⁸ peut représenter des radicaux bifonctionnels aromatiques et linéaires ou cycliques, saturés ou insaturés, avec des nombres de carbone de C₂ à C₂₂
T : un composé parmi le groupe constitué des composés organyle sulfonés aromatiques, aliphatiques ou cycloaliphatiques, renfermant au moins deux groupes acyle terminaux
R¹ : peut représenter un lithium et/ou un sodium et éventuellement d'autres contre-ions, par exemple, un potassium, un magnésium, un calcium, un ammonium, un monoalkylammonium, un dialkylammonium, un trialkylammonium ou un tétraalkylammonium, les positions alkyle des amines étant occupées, indépendamment les unes des autres, par des radicaux alkyle en C₁ à C₂₂ et de 0 à 3 groupes hydroxy
R² : un radical moléculaire choisi parmi les groupes constitués
- des fonctions amino aromatiques, aliphatiques ou cycloaliphatiques (-NH-R⁵, -NR⁵₂ où R⁵ peut représenter un radical alkyle ou aryle en C₁ à C₂₂)
- des groupes acides monocarboxyliques, aromatiques, aliphatiques ou cycloaliphatiques : (-COOR⁶ où R⁶ représente un radical alkyle ou aryle en C₁ à C₂₀₀)
- des radicaux organyle aromatiques, aliphatiques
ou cycloaliphatiques pontés par l'intermédiaire de fonctions éther : (-O-R⁵)
- des composés polyalcoxy à pontage par l'intermédiaire de fonctions éther de formule
-O-[R⁷-O]_{q}-[R⁸-O]ᵣY
les radicaux R⁷ et R⁸ représentant avantageusement des radicaux alkyle ayant un nombre de carbones de C₂ à C₂₂, les deux radicaux n'étant pas nécessairement différents ; le radical Y pouvant être aussi bien un hydrogène que de nature aliphatique en C₁ à C₂₂ ; pour les coefficients q et r : q + r ≥ 1.
- des radicaux organyle sulfonés mono- ou polyéthoxylés à pontage par l'intermédiaire de fonctions éther ou de préférence leurs sels de métaux alcalins ou alcalinoterreux tels que, par exemple, avantageusement **caractérisés par** la formule structurale générique-(O-CH₂-CH₂)ₛ-SO₃R¹
dans laquelle s ≥ 1, et s pouvant en outre être choisi de telle sorte que les poids moléculaires moyens indiqués précédemment des constituants de chaîne principale utilisés soient obtenus.

4. Polymères en peigne selon la revendication 1, **caractérisés en ce que** leurs poids moléculaires moyens sont avantageusement compris entre 200 et 2 000 000 g/mol, particulièrement avantageusement entre 200 et 100 000 g/mol, la plage de 1 000 à 30 000 g/mol étant de préférence utilisée, tout particulièrement avantageusement de 5 000 à 15 000 g/mol.

5. Compositions de traitement capillaire présentant une teneur efficace en un ou plusieurs polymères en peigne selon l'une quelconque des revendications 1 à 4.
